# EUROPEAN PATENT APPLICATION

(11) **EP 1 946 759 A1**
(43) Date of publication of application: **23.07.2008**
(21) Application number: 06811510.4
(22) Date of filing: 10.10.2006
(51) Int. Cl.: A61K 31/485, A61P 1/08, C07D 489/08

(54) **THERAPEUTIC AGENT FOR NAUSEA AND/OR VOMITING**

(30) Priority: 11.10.2005 JP 2005296025
(71) Applicant: Toray Industries, Inc., Tokyo, 103-8666 (JP)
(72) Inventor: SUZUKI, Tsutomu, Kanagawa 235-0019 (JP); ISHIHARA, Yasunobu, Osaka 553-0002 (JP)
(74) Representative: Kador & Partner
(86) International application number: PCT/JP2006/320197
(87) International publication number: WO 2007/043518

(57) **Abstract**

A therapeutic and/or prophylactic agent for nausea and/or vomiting is provided. A therapeutic and/or prophylactic agent for nausea and/or vomiting, comprising a compound of the Formula (I) or a pharmaceutically acceptable salt or solvate thereof: 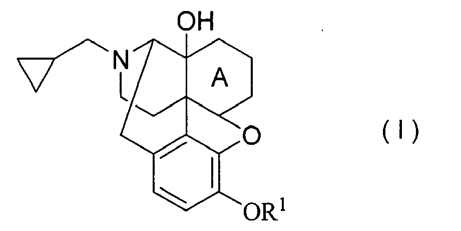 (wherein 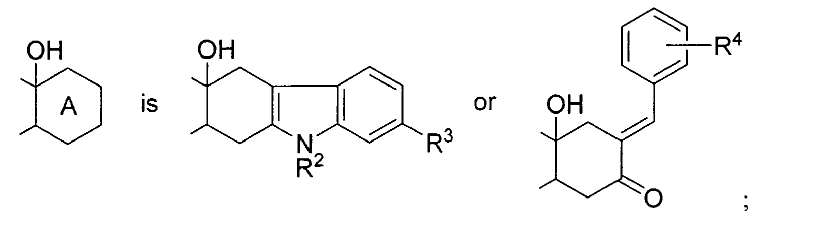 R¹ and R² are each independently hydrogen or lower alkyl;
R³ is hydrogen or lower alkoxycarbonyl; and
R⁴ is hydrogen or lower alkyl;

with the proviso that all of R¹ to R³ are not simultaneously hydrogen; and R¹ and R⁴ are not simultaneously hydrogen).

## Description

### Technical Field

The present invention relates to a therapeutic and/or prophylactic agent for nausea and/or vomiting, especially the nausea and/or vomiting induced by a compound having an opioid receptor (e.g., opioid µ receptor) agonist activity.

### Background Art

Opioid µ receptor agonists such as morphine are used for patients with pain as very effective analgesics. However, they induce strong vomiturition, nausea, vomiting, urinary retention, itching and so on as side effects. Although various antiemetics are clinically used, none of them exhibits sufficient effect, so that an excellent therapeutic or prophylactic agent for nausea and/or vomiting is demanded for improvement in QOL of the patients.
Patent Literature 1 discloses that compounds analogous to the compounds of the present invention are effective for the therapy or prophylaxis of vomiturition or vomiting induced by opioid µ agonists. However, the compounds of the present invention are not concretely described.
Patent Literatures 2-5 and Non-patent Literature 1 disclose the compounds of the present invention or compounds analogous thereto. However, it is not described that the compounds of the present invention can be a particularly excellent therapeutic and/or prophylactic agent for nausea and/or vomiting.
Patent Literature 1: WO2004/007503 A
Patent Literature 2: WO94/07896 A
Patent Literature 3: WO89/00995 A
Patent Literature 4: WO95/31463 A
Patent Literature 5: WO95/13071 A
Non-patent Literature 1: Journal of Medicinal Chemistry 41, 4177-4180 (1998)

### Disclosure of the Invention

### Problems Which the Invention Tries to Solve

A therapeutic and/or prophylactic agent for nausea and/or vomiting, especially the nausea and/or vomiting induced by a compound having an opioid µ receptor agonist activity is provided.

### Means for Solving the Problems

The present invention provides:
(1) A therapeutic and/or prophylactic agent for nausea and/or vomiting, comprising a compound of the Formula (I) or a pharmaceutically acceptable salt or solvate thereof: 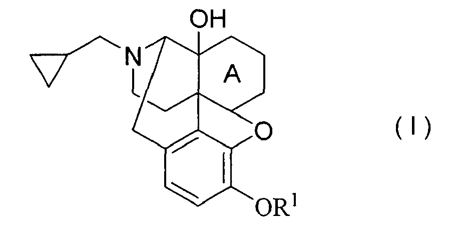 (wherein 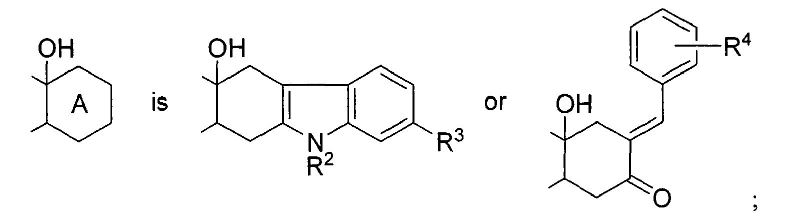
   R¹ and R² are each independently hydrogen or lower alkyl;
   R³ is hydrogen or lower alkoxycarbonyl; and
   R⁴ is hydrogen or lower alkyl;
   with the proviso that all of R¹ to R³ are not simultaneously hydrogen; and R¹ and R⁴ are not simultaneously hydrogen);
(2) The therapeutic and/or prophylactic agent according to (1) described above, which comprises the compound wherein R¹ is hydrogen, R² is methyl and R³ is hydrogen; the compound wherein R¹ is hydrogen, R² is hydrogen and R³ is ethoxycarbonyl; or the compound wherein R¹ is hydrogen and R⁴ is methyl; or a pharmaceutically acceptable salt or solvate thereof;
(3) The therapeutic and/or prophylactic agent according to (1) or (2) described above, wherein the nausea and/or vomiting is one induced by a compound having an opioid receptor agonist activity.
(4) The therapeutic and/or prophylactic agent according to (3) described above, wherein the compound having the opioid receptor agonist activity is morphine, oxycodone or a pharmaceutically acceptable salt or solvate thereof;
(5) Use of the compound of Formula (1) or the pharmaceutically acceptable salt or solvate thereof recited in (1) described above, for the production of a pharmaceutical for the therapy and/or prophylaxis of nausea and/or vomiting;
(6) Use of the compound of Formula (1) or the pharmaceutically acceptable salt or solvate thereof recited in (1) described above, for the production of a pharmaceutical for the therapy and/or prophylaxis of nausea and/or vomiting induced by a compound having an opioid receptor agonist activity;
(7) A therapeutic and/or prophylactic method for nausea and/or vomiting, the method comprising administering the compound of Formula (1) or the pharmaceutically acceptable salt or solvate thereof recited in (1) described above;
(8) A therapeutic and/or prophylactic method for nausea and/or vomiting induced by a compound having an opioid receptor agonist activity, the method comprising administering the compound of Formula (1) or the pharmaceutically acceptable salt or solvate thereof recited in (1) described above; and
(13) An analgesic comprising a compound having an opioid receptor agonist activity, and the compound of Formula (1) or the pharmaceutically acceptable salt or solvate thereof recited in (1) described above, in an amount effective for the therapy and/or prophylaxis of the nausea and/or vomiting induced by the administration of the compound having an opioid receptor agonist activity.

### Effects of the Invention

The compounds (I) of the present invention have a therapeutic and/or prophylactic effect for the nausea and/or vomiting, especially the nausea and/or vomiting induced by a compound having an opioid receptor agonist activity, so that they are useful as an agent for ameliorating the side effects of a patient to whom a compound having an opioid µ receptor agonist activity is to be administered or is being administered.

### Best Mode for Carrying out the Invention

As used herein, "halogen" includes fluorine, chlorine, bromine and iodine.
"Lower alkyl" includes C₁-C₁₀, preferably C₁-C₆, more preferably C₁-C₃ linear or branched alkyl groups. Examples thereof include methyl, ethyl, *n*-propyl, isopropyl, *n*-butyl, isobutyl, *sec*-butyl, *tert*-butyl, *n*-pentyl, isopentyl, neopentyl, hexyl, isohexyl, *n*-heptyl, isoheptyl, *n*-octyl, isooctyl, *n*-nonyl, *n*-decyl and the like. Preferred is methyl or ethyl.
The lower alkyl moiety in "lower alkoxycarbonyl" is the same as the "lower alkyl" described above. Preferred is methoxycarbonyl or ethoxycarbonyl.

As used herein, "solvate" includes, for example, solvates with organic solvents, hydrates and the like. When a hydrate is formed, the compound may be coordinated with an optional number of water molecule(s).
The compounds (I) include pharmaceutically acceptable salts thereof. Examples thereof include salts with an alkaline metal (such as lithium, sodium or potassium), alkaline earth metal (such as magnesium or calcium), ammonium, an organic base or an amino acid; and salts with an inorganic acid (such as hydrochloric acid, sulfuric acid, nitric acid, hydrobromic acid, phosphoric acid or hydroiodic acid) or an organic acid (such as acetic acid, citric acid, lactic acid, tartaric acid, oxalic acid, maleic acid, fumaric acid, mandelic acid, glutaric acid, malic acid, benzoic acid, phthalic acid, benzenesulfonic acid, *p-*toluenesulfonic acid, methanesulfonic acid or ethanesulfonic acid). Especially preferred are salts with hydrochloric acid, phosphoric acid, tartaric acid, methanesulfonic acid or the like. These salts may be formed by a conventional method.
The compounds (I) are not restricted to a particular isomer, but include all of the possible isomers and racemic mixtures.

The compounds (I) of the present invention can be produced by the methods described in the above-described Patent Literatures 1-5 and Non-patent Literature 1.
"Nausea and/or vomiting" means the vomiturition, nausea and/or vomiting, especially those induced by taking a compound having an opioid receptor (e.g., opioid µ receptor) agonist activity. Specific examples of the "compound having an opioid receptor agonist activity" include morphine, oxycodone, fentanyl, methadone, codeine, dihydrocodeine, hydromorphone, levorphanol, meperidine, propoxyphene, dextropropoxyphene, tramadol and pharmaceutically acceptable salts and solvates thereof. The therapeutic and/or prophylactic agent according to the present invention is especially effective when the compound having an opioid receptor agonist activity is morphine or oxycodone, or a pharmaceutically acceptable salt or solvate thereof.

The compound of the present invention is effective for the therapy and/or prophylaxis of nausea and/or vomiting caused by acute dyspepsia, acute alcoholism, food poisoning, common cold, gastric ulcer, duodenal ulcer, gastric cancer, intestinal obstruction, appendicitis, peritonitis, cholelithiasis, hepatitis, hepatitis, encephalitis, meningitis, increased intracranial pressure, head injury, motion sickness, hyperemesis gravidarum, side effects of chemotherapy, side effects of anticancer drug or the like, side effects of radiotherapy, gastrointestinal transit disorder due to compression or stenosis of digestive tract or due to postoperational intestinal adhesion, or increase in intracranial pressure due to brain tumor, cerebral hemorrhage, meningitis, radiation to the brain, or the like.
As is apparent from the test examples described below, since the brain penetration of the compound is low, the compound exhibits high amelioration effect against the nausea and/or vomiting induced by the opioid receptor agonist without substantially inhibiting the analgesic action of the compound having the opioid receptor agonist activity administered to patients suffering from a disorder accompanying pain (e.g., cancer pain (pain due to bone metastasis, compression of nerve, intracranial hypertension, soft tissue infiltration or contraction of muscle, pain of viscus, muscle or fascia, pain of the vicinity of waist or shoulder joint, postoperative chronic pain), AIDS or the like).
The compounds (I) also have characteristics such as a strong opioid δ receptor antagonist activity, high receptor selectivity, high oral absorption, low toxicity, high stability in human plasma and high bioavailability, so that they are very useful as a pharmaceutical.

When administering the compound of the present invention against the nausea and/or vomiting induced by an opioid receptor agonist, the compound of the present invention may be administered before, after or simultaneously with the administration of the compound having the opioid receptor agonist activity.
The interval between the administrations of these two drugs is not restricted. For example, in cases where the compound of the present invention is administered after administration of the compound having the opioid receptor agonist activity, the compound of the present invention more effectively acts if it is administered immediately after or within about 3 days after, preferably immediately after or within about 1 day after the administration of the opioid receptor agonist. In cases where the compound of the present invention is administered before the administration of the opioid receptor agonist, the compound of the present invention more effectively acts if it is administered immediately before or within 1 day before, preferably immediately before or within 12 hours before the administration of the opioid receptor agonist.
When the compound of the present invention is administered as a therapeutic and/or prophylactic agent for nausea and/or vomiting, it may be administered in combination with other therapeutic and/or prophylactic agent(s) for nausea and/or vomiting. For example, it may be administered in combination with ondansetron hydrochloride, an adrenocorticosteroid (such as methylprednisolone, prednisolone or dexamethasone), prochlorperazine, haloperidol, timiperone, perphenazine, metoclopramide, domperidone, scopolamine, chlorpromazine hydrochloride, droperidol or the like.
The compound of the present invention may be administered in the form of a combination drug with a compound(s) having the opioid receptor agonist activity, or in the form of a combination drug with other therapeutic and/or prophylactic agent(s) for nausea and vomiting and/or therapeutic and/or prophylactic agent(s) for constipation.
When the compound of the present invention is administered to human, it may be administered orally in the form of a powder, granules, tablet, capsule, ball, liquid or the like; or parenterally in the form of an injection solution, suppository, transdermal formulation, inhalant or the like. An effective amount of the compound may be admixed with a pharmaceutical additive(s) such as a vehicle, binder, wetting agent, disintegrant, lubricant and the like, as required, to form a pharmaceutical formulation.
The compound of the present invention may be formulated into a mixture with the compound having the opioid receptor agonist activity and/or other therapeutic and/or prophylactic agent(s) for nausea and/or vomiting and/or pharmaceutical additive(s) as necessary.
Although the dose differs depending on the conditions of the disease, administration route, age and body weight of the patient, when the compound is orally administered to an adult, the dose is usually 0.1 µg to 1 g/day, preferably 0.01 to 200 mg/day, and when the compound is parenterally administered, the dose is usually 1 µg to 10 g/day, preferably 0.1 to 2 g/day.

The present invention is now described in more detail referring to examples and test examples. However, the present invention is not restricted thereto.

### Examples

### Test Example 1 Influence on Nausea and Vomiting Induced by Morphine

### 1) Test Substances

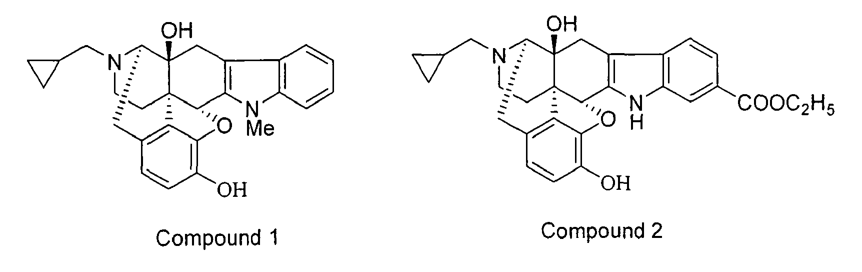

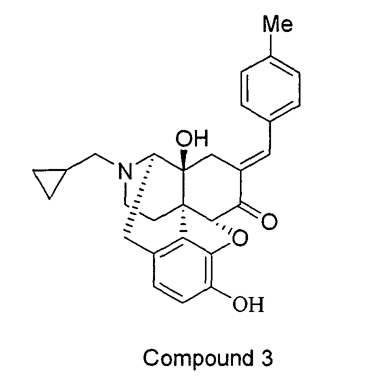

### 2) Testing Method

About 30 minutes after feeding, ferrets were transferred to observation cages, and the test substances were administered, respectively. Each test substance was dissolved in 5% xylitol, and administered at a dose of 5 mg/kg. Thirty minutes after administration of the test solution, 0.6 mg/kg of morphine was subcutaneously administered at a volume of 1 mL/kg, and the symptoms of vomiting were visually observed until 30 minutes after the administration of morphine.
The appearance frequency, latent time (the time from the administration of morphine to the first appearance of the symptom) and duration (the time from the first vomiting to the final vomiting) of nausea (rhythmical contractile motion of abdomen), of vomiting (vomiting behavior excreting vomit or analogous behavior thereof) and of the total of nausea and vomiting were counted, respectively.
In cases where vomiting did not appear, the latent time was counted as 30 minutes which was the maximum value at the end of observation, and in cases where the duration of vomiting was less than 1 minute, the duration was counted as 1 minute for convenience.
The results are shown in Table 1.

**Table 1**

| | | Dose (mg/kg) | Number of Individuals Presenting Symptoms (Number of Individuals Presenting Symptoms/Number of Cases) | Latent Time (min) | Duration (min) | Appearance Frequency |
|---|---|---|---|---|---|---|
| Nausea | Control^{a)} | - | 6/6 | 9±3 | 9±3 | 34±11 |
| | Compound 1 | 5 | 0/6## | 30±0** | 0±0** | 0±0** |
| | Compound 2 | 5 | 0/6## | 30±0** | 0±0** | 0±0** |
| | Compound 3 | 5 | 0/6## | 30±0** | 0±0** | 0±0** |
| Vomiting | Control^{a)} | - | 4/6 | 15±5 | 7±2 | 3±1 |
| | Compound 1 | 5 | 0/6 | 30±0* | 0±0* | 0±0** |
| | Compound 2 | 5 | 0/6 | 30±0* | 0±0* | 0±0** |
| | Compound 3 | 5 | 0/6 | 30±0* | 0±0* | 0±0** |
| Nausea + Vomiting | Control ^{a)} | - | 6/6 | 9±3 | 9±3 | 36±12 |
| | Compound 1 | 5 | 0/6## | 30±0** | 0±0** | 0±0** |
| | Compound 2 | 5 | 0/6## | 30±0** | 0±0** | 0±0** |
| | Compound 3 | 5 | 0/6## | 30±0** | 0±0** | 0±0** |

| | | | | | | |
|---|---|---|---|---|---|---|
| Each value is expressed in terms of mean ± standard error. a): 5% xylitol, 0.5 mL/kg, s.c. ##: This means that the significant difference from the control was P<0.01 (by Fisher's exact probability test) * and **: These means that the significant difference from the control was P<0.05 and P<0.01 (Dunnett's test), respectively. | | | | | | |

As seen from these results, the compounds (I) show an antagonistic action against nausea and vomiting induced by the administration of morphine.

### Test Example 2 Test of Brain Penetration

1) Test Compounds
   The above-described Compounds 2 and 3 were used.
2) Animals Used
   Crj:CD(SD)IGS male rats of 7-8 weeks of age
3) Administered Test Solution
   Each of the test compounds in an amount of 90 mg was suspended in 0.5% methylcellulose 1500 cp to a total volume of 6 mL.
4) Collected Samples: blood and brain
5) Measurement of Plasma Concentration
   Each test compound was orally administered to non-fasting rats (30 mg/2 mL/kg, n=3), and total blood was collected from the lower aorta 2 hours after the administration. An aliquot of the blood was centrifuged (3000 rpm, 10 minutes, 4°C), and the concentration of unchanged compound in the obtained blood plasma was measured by LC/MS/MS method.
6) Measurement of Concentration in Brain Tissue
   After the above-described collection of the total blood, the brain was enucleated from each rat, and homogenized to prepare a 25% homogenate, followed by measurement of the concentration of unchanged compound by LC/MS/MS method.
7) Results
   From the obtained concentrations, Kp values were determined. The results are shown in Table 2.

**Table 2**

| Test compound | Kp |
|---|---|
| Compound 2 | 0.18 |
| Compound 3 | 0.46 |

### Reference Example 1 Test of Brain Penetration

1) Test Compound
   NTI (naltrindole) obtained by the method described in Reference Example 1 of the above-described Patent Literature 1 was used.
2) Animals Used
   Jcl-SD male rats of 7 weeks of age
3) Administered Test Solution
   The test compound in an amount of 1.0 mg was dissolved in 0.8 mL of N,N-dimethylacetamide, and 0.8 mL of propylene glycol was added thereto.
4) Collected Samples: blood and brain
5) Measurement of Plasma Concentration
   The test compound was intravenously administered to non-fasting rats (0.5 mg/0.8 mL/kg, n=2) through the tail vein, and total blood was collected from the lower aorta 15 minutes after the administration. An aliquot of the blood was centrifuged (3000 rpm, 10 minutes, 4°C), and the concentration of unchanged compound in the obtained blood plasma was measured by LC/MS/MS method.
6) Measurement of Concentration in Brain Tissue
   After the above-described collection of the total blood, the brain was enucleated from each rat, and homogenized to prepare a 25% homogenate, followed by measurement of the concentration of unchanged compound by LC/MS/MS method.
7) Results
   From the obtained concentrations, Kp values were determined. As a result, the Kp value of NIT was 1.30.

As seen from these results, brain penetration of the compounds (I) is low, and particularly, it is lower than that of NTI described in the above-described Patent Literature 1. Therefore, the compounds (I) of the present invention can ameliorate the nausea and vomiting which are the side effects of the opioid receptor agonist without inhibiting the analgesic action of the opioid receptor agonist.

### Formulation Example 1

Granules containing the following components are prepared:

| | | |
|---|---|---|
| Component | Compound of Formula (I) | 10 mg |
| | Lactose | 700 mg |
| | Corn starch | 274 mg |
| | HPC-L | 16 mg |
| | | 1000 mg |

The compound of the Formula (I) and lactose are made to pass through a 60-mesh sieve. Corn starch is made to pass through a 120-mesh sieve. These are mixed in a V-blender. To the mixed powder, aqueous HPC-L (low viscosity hydroxypropylcellulose) solution is added, and the obtained mixture is kneaded, granulated (extrusion granulation, pore diameter 0.5-1 mm) and dried. The obtained dried granules are sieved through a vibrating screen (12/60-mesh) to obtain granules.

### Formulation Example 2

Granules for preparation of capsules, containing the following components are prepared:

| | | |
|---|---|---|
| Component | Compound of Formula (I) | 15 mg |
| | Lactose | 90 mg |
| | Corn starch | 42 mg |
| | HPC-L | 3 mg |
| | | 150 mg |

The compound of the Formula (I) and lactose are made to pass through a 60-mesh sieve. Corn starch is made to pass through a 120-mesh sieve. These are mixed, and HPC-L solution is added to the mixture. The obtained mixture is kneaded, granulated and dried. The obtained dried granules are subjected to size selection and then 150 mg aliquot thereof is encapsulated in a No. 4 hard gelatin capsule.

### Formulation Example 3

Tablets containing the following components are prepared:

| | | |
|---|---|---|
| Component | Compound of Formula (I) | 10 mg |
| | Lactose | 90mg |
| | Microcrystalline cellulose | 30mg |
| | CMC-Na | 15 mg |
| | Magnesium stearate | 5 mg |
| | | 150 mg |

The compound of the Formula (I), lactose, microcrystalline cellulose and CMC-Na (carboxymethylcellulose sodium salt) are made to pass through a 60-mesh sieve, and mixed. The mixed powder is mixed with magnesium stearate to obtain mixed powder for preparing tablets. This mixed powder is directly compressed to obtain tablets each weighing 150 mg.

### Formulation Example 4

The following components were mixed under heat, and the mixture was sterilized to obtain an injection solution.

| | | |
|---|---|---|
| Component | Compound of Formula (I) | 3 mg |
| | Nonionic surfactant | 15 mg |
| | Purified water for injection | 1 ml |

### Industrial Availability

The compounds of the present invention can be a pharmaceutical useful as a therapeutic and/or prophylactic agent for nausea and/or vomiting.

## Claims

1. A therapeutic and/or prophylactic agent for nausea and/or vomiting, comprising a compound of the Formula (I) or a pharmaceutically acceptable salt or solvate thereof: 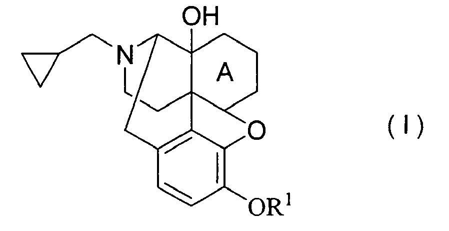 (wherein 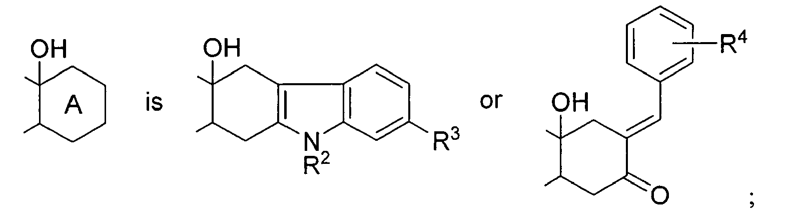
R¹ and R² are each independently hydrogen or lower alkyl;
R³ is hydrogen or lower alkoxycarbonyl; and
R⁴ is hydrogen or lower alkyl;
with the proviso that all of R¹ to R³ are not simultaneously hydrogen; and R¹ and R⁴ are not simultaneously hydrogen).

2. The therapeutic and/or prophylactic agent according to claim 1, which comprises the compound wherein R¹ is hydrogen, R² is methyl and R³ is hydrogen; the compound wherein R¹ is hydrogen, R² is hydrogen and R³ is ethoxycarbonyl; or the compound wherein R¹ is hydrogen and R⁴ is methyl; or a pharmaceutically acceptable salt or solvate thereof.

3. The therapeutic and/or prophylactic agent according to claim 1 or 2, wherein said nausea and/or vomiting is one induced by a compound having an opioid receptor agonist activity.

4. The therapeutic and/or prophylactic agent according to claim 3, wherein said compound having the opioid receptor agonist activity is morphine, oxycodone or a pharmaceutically acceptable salt or solvate thereof.
